# EUROPEAN PATENT SPECIFICATION

(11) **EP 2 588 979 B1**
(45) Date of publication and mention of the grant of the patent: **27.04.2016**
(21) Application number: 10798112.8
(22) Date of filing: 24.12.2010
(51) Int. Cl.: G06F 19/00, H04L 29/08

(54) **MOBILE POINT OF CARE TESTING UNIT WITH DYNAMIC PROFILE AND RELATED METHOD**
MOBILE POINT-OF-CARE-TESTING EINHEIT MIT DYNAMISCHEM PROFIL UND ZUGEHÖRIGES VERFAHREN
UNITÉ D'ANALYSE AU CHEVET DU PATIENT MOBILE À PROFIL DYNAMIQUE ET PROCÉDÉ ASSOCIÉ

(43) Date of publication of application: 08.05.2013
(73) Proprietor: F.Hoffmann-La Roche AG, 4070 Basel (CH); Roche Diagnostics GmbH, 68305 Mannheim (DE)
(72) Inventor: SABARÍS VILAS, Joaquín, E-08213 Polinyà del Vallés (Barcelona) (ES)
(74) Representative: ZBM Patents - Zea, Barlocci & Markvardsen
(86) International application number: PCT/EP2010/070725
(87) International publication number: WO 2012/084063

(56) References cited:
- EP-A1- 1 862 112
- MDNEIL A HALPERNA AND MDTRICIA BRENTJENSB: "POINT OF CARE TESTING INFORMATICS: The Critical Care-Hospital Interface", CRITICAL CARE CLINICS, W.B. SAUNDERS, US, vol. 15, no. 3, 1 July 1999 (1999-07-01), pages 577-591, XP008132749, ISSN: 0749-0704, DOI: 10.1016/S0749-0704(05)70072-5

## Description

The present invention relates to a method for obtaining a dynamic profile of a mobile POCT unit from a central POCT manager; and to a mobile POCT unit and a computer program suitable for carrying out such a method.

The present invention also relates to a method for providing a dynamic profile of a mobile POCT unit; and to a central POCT manager and a computer program suitable for carrying out such a method.

### BACKGROUND ART

Point of Care Testing (POCT) refers to a set of techniques to be performed outside the laboratory as decentralized diagnosis. Justification of POCT is based on immediate response times to obtain improved results for patient diagnosis and treatment. The main weaknesses of POCT are derived from the lack of security in the obtained results and the consequent undefined responsibility determination. Thanks to the facilities of industry developments on systems based on micro/nanotechnology and new diagnostic applications for specific analytical parameters, POCT has proliferated towards a diverse mass of equipments very difficult to control.

POCT analyzers are very easy to use and, thus, they may be operated by personnel with no laboratory experience, in which case, deficiencies on responsibility determination arise because operators cannot assume the responsibility of security/reliability in the obtained results.

Some POCT analyzers comprise modules dedicated to autonomous quality controls, for example Auto-QC modules, being focused to ensure technical reliability according to a local approach, that is to say, the results of said quality controls are usually verified by the operator without intervention of laboratory experts. Thus, undefined responsibility determination still remains despite these quality control modules.

POCT analyzers have progressively reached higher portability, this fact causing the existence of diverse mass of POCT analyzers difficult to control according to a centralized approach, for example, by a central laboratory.

Micro and Nanotechnology have made possible very small POCT analyzers, so that medical centres usually have big numbers of POCT analyzers distributed within the medical centre, said massive distribution aggravating the abovementioned undefined responsibility determination.

The undefined responsibility determination may be attenuated with a lot of experts on POCT analyzers continuously checking in situ and one-to-one the technical status of the analyzers, but this solution is inefficient, because a lot of experts are required, and inflexible, due to the in situ and one-to-one checking.

Different software applications for remotely managing technical status of POCT analyzers are known, as for example Roche's cobas® IT 1000 application, said applications being only focused to the individual technical maintenance of the POCT analyzers, requiring a permanent attention of several technicians on one or more screens showing the technical status of the POCT analyzers. These applications have the drawback of still being inefficient, since permanent attention of several technicians is required, and the drawback of being inflexible, since only one-to-one technical maintenance of POCT analyzers is supported.

Moreover, these applications for remotely managing technical status of POCT analyzers provide data very complex to understand for personnel without laboratory experience and, besides, no perceptible signal indicating a "failed" status for some of the analyzers is generated for the user of the analyzer. Only the technicians in charge of the application are notified.

EP1862112 discloses systems, client computing devices, server computing devices, and methods for accessing medical devices, providing remote access to medical devices and/or remotely accessing medical devices. Client computing devices may utilize protocol components that may be obtained from a server computing device via a network to communicate with medical devices in a communications protocol supported by the medical device.

In conclusion, the currently known technologies supporting remote management of POCT analyzers have the drawbacks of being inefficient and inflexible.

### SUMMARY OF THE INVENTION

There thus still exists a need for improved methods, devices and computer programs solving the drawbacks of the current inefficient and inflexible technologies supporting the remote management of POCT analyzers. The object of the present invention is to fulfil such a need.

Said object is achieved with the methods, computer programs and systems claimed in the appended claims which define the scope of the invention. In particular, it is provided a computer-implemented method according to independent claim 1, a computer program according to independent claim 7, a computer-implemented method according to independent claim 8, a mobile POCT unit according to independent claim 14, a central POCT manager system according to independent claim 15, and a network of POCT units according to independent claim 16.

In a first aspect, the present invention provides a mobile POCT unit with dynamic profile, comprising:
- computing means for wirelessly identifying a set of POCT analyzers;
- computing means for sending, to the central POCT manager system, a POCT analyzer identity for each identified POCT analyzer;
- computing means for receiving, from the central POCT manager system, the dynamic profile of the mobile POCT unit comprising a linked/unlinked status for each identified POCT analyzer, said linked status having been determined by the central POCT manager system for each identified POCT analyzer having related quality data satisfying predefined quality requirements, and representing that the identified POCT analyzer is operative for the mobile POCT unit; and said unlinked status having been determined by the central POCT manager system for each identified POCT analyzer having related quality data not satisfying predefined quality requirements, and representing that the identified POCT analyzer is not operative for the mobile POCT unit.

This mobile POCT unit with dynamic profile allows an efficient way of controlling POCT analyzers, because there is no necessity of permanent attention of experts on POCT analyzers continuously checking the technical status of the analyzers.

Furthermore, this mobile POCT unit with dynamic profile allows a very flexible way of controlling POCT analyzers, because operators (e.g. doctors, nursery, clinicians or physicians) of the mobile unit may obtain exclusivity for using new POCT analyzers, so that the profile of the POCT unit may be dynamically adapted to the changing necessities of the doctor. For example, if the doctor requires new analytical parameters not present in any of the analyzers currently linked to the POCT unit, the profile of the POCT unit may be extended by obtaining new linked analyzers comprising said new analytical parameters.

An example of the flexibility provided by this mobile POCT unit is the possibility of linking the POCT analyzers from an ambulance when said ambulance arriving to a hospital with a patient that, for example, has been injured in an accident. Between the time of the accident and the time of arrival to the hospital, the medical personnel of the ambulance have been accumulating analytical data obtained through the POCT analyzers, said data being temporally stored in the own memory of the analyzers. Then, once the analyzers have been linked to the mobile POCT unit, the accumulated analytical data is available in the mobile POCT unit, which may be operated as a continuation of the treatments that the patient has received in the ambulance. Then, with the mobile POCT unit of the invention a new medical paradigm arises: not only the patient is admitted to the hospital, but the patient and the POCT analyzers used for monitoring the patient in the ambulance, included the analytical data obtained through said analyzers.

Said "transfer" of analyzers from the ambulance to the mobile POCT unit is named SWAPING and allows controlling the state of the patient from the first moment in the stage of the accident, even in zones very distant from a medical centre of reference, till the end of the care process in the medical centre. In other words, the same analyzers may be used for monitoring the patient from the very first moment to the very last moment.

In a second aspect, the present invention provides a central POCT manager system, comprising:
- computing means for receiving from the mobile POCT unit a set of POCT analyzer identities;
- computing means for determining a dynamic profile of the mobile POCT unit;
- computing means for sending, to the mobile POCT unit, the dynamic profile of the mobile POCT unit,
the dynamic profile comprising a linked/unlinked status of each identified POCT analyzer, and the computing means for determining the dynamic profile of the mobile POCT unit comprising:
- computing means for determining the linked status for each identified POCT analyzer having related quality data satisfying predefined quality requirements, said linked status representing that the identified POCT analyzer is operative for the mobile POCT unit;
- computing means for determining the unlinked status for each identified POCT analyzer having related quality data not satisfying predefined quality requirements, said unlinked status representing that the identified POCT analyzer is not operative for the mobile POCT unit.

The central POCT manager accepts linking of an analyzer to a mobile POCT unit only in case of the analyzer having quality data satisfying quality requirements, so the personnel controlling said quality parameters (e.g. the central laboratory) has the validation responsibility, that is to say, any result obtained from a linked analyzer may be considered validated by the central laboratory because said linked analyzer satisfies the quality requirements defined by the central laboratory. Therefore, this central POCT manager allows an automatic centralized control of POCT analyzers with a defined responsibility determination and without requiring permanent attention of laboratory experts.

This central POCT manager permits an indirect control of POCT analyzers, since the central POCT manager may only interact with mobile POCT units which may be considered as concentrators of POCT analyzers in a way that any analyzer is under strict control of the central POCT manager when said analyzer is linked to a POCT unit. This indirect control approach has also the advantage of decreasing interferences of wireless signals in comparison with a central POCT manager directly interacting with the POCT analyzers. Each mobile POCT unit may only interact locally with the surrounding analyzers, so wireless signals between a mobile POCT unit and the surrounding analyzers may be short-range signals, whereas in a direct control approach, without intermediate POCT units, the wireless signals for identifying analyzers should be signals of longer range, said signals of longer range highly increasing the possibilities of interferences in relation to the short-range signals.

In conclusion, the central POCT manager of the invention has the main advantages of allowing a more efficient and flexible control of POCT analyzers.

In a third aspect of the invention, it is provided a computer-implemented method for obtaining a dynamic profile of a mobile POCT unit from a central POCT manager system, said method comprising the mobile POCT unit continuously repeating the following steps according to a predefined frequency:
- wirelessly identifying a set of POCT analyzers;
- sending, to the central POCT manager system, a POCT analyzer identity for each identified POCT analyzer;
- receiving, from the central POCT manager system, the dynamic profile of the mobile POCT unit comprising a linked/unlinked status for each identified POCT analyzer, said linked status having been determined by the central POCT manager system for each identified POCT analyzer having related quality data satisfying predefined quality requirements, and representing that the identified POCT analyzer is operative for the mobile POCT unit; and said unlinked status having been determined by the central POCT manager system for each identified POCT analyzer having related quality data not satisfying predefined quality requirements, and representing that the identified POCT analyzer is not operative for the mobile POCT unit.

This method for obtaining a dynamic profile of a mobile POCT unit from a central POCT manager allows to the mobile POCT unit of the invention to act as described before. Then the advantages commented in relation to the mobile POCT unit may also be attributed to this method.

In a fourth aspect, the present invention also provides a computer program product comprising program instructions for causing a computer to perform, when said program is run on said computer, a computer-implemented method for obtaining a dynamic profile of a mobile POCT unit from a central POCT manager. The invention also relates to such a computer program product embodied on a storage medium (for example, a CD-ROM, a DVD, a USB drive, on a computer memory or on a read-only memory) or carried on a carrier signal (for example, on an electrical or optical carrier signal).

In a fifth aspect of the invention, it is provided a method for providing a dynamic profile of a mobile POCT unit, said method comprising:
- receiving from the mobile POCT unit a set of POCT analyzer identities;
- determining the dynamic profile of the mobile POCT unit;
- sending, to the mobile POCT unit, the dynamic profile of the mobile POCT unit,
the dynamic profile comprising a linked/unlinked status of each identified POCT analyzer, and determining the dynamic profile of the mobile POCT unit comprising:
- determining the linked status for each identified POCT analyzer having related quality data satisfying predefined quality requirements, said linked status representing that the identified POCT analyzer is operative for the mobile POCT unit;
- determining the unlinked status for each identified POCT analyzer having related quality data not satisfying predefined quality requirements, said unlinked status representing that the identified POCT analyzer is not operative for the mobile POCT unit.

This method for providing a dynamic profile of a mobile POCT unit allows to the central POCT manager of the invention to act as described before. Then the advantages commented in relation to the central POCT manager may also be attributed to this method.

In a sixth aspect, the present invention also provides a computer program product comprising program instructions for causing a computer to perform the method for providing a dynamic profile of a mobile POCT unit. The invention also relates to such a computer program product embodied on a storage medium (for example, a CD-ROM, a DVD, a USB drive, on a computer memory or on a read-only memory) or carried on a carrier signal (for example, on an electrical or optical carrier signal).

Optional and advantageous features of the device and methods are set out in the dependent claims.

Additional objects, advantages and features of embodiments of the invention will become apparent to those skilled in the art upon examination of the description, or may be learned by practice of the invention.

### BRIEF DESCRIPTION OF THE DRAWINGS

Particular embodiments of the present invention will be described in the following by way of non-limiting examples, with reference to the appended drawings, in which:
Figure 1 is a schematic representation of a mobile POCT unit with dynamic profile, according to an embodiment of the invention;
Figure 2 is a schematic representation of a network of mobile POCT units with dynamic profile, according to an embodiment of the invention; and
Figure 3 is a schematic representation of a mobile POCT unit and its related area of influence, according to an embodiment of the invention.

### DETAILED DESCRIPTION OF EMBODIMENTS OF THE INVENTION

In the following descriptions, numerous specific details are set forth in order to provide a thorough understanding of the present invention. It will be understood, however, to one skilled in the art, that the present invention may be practiced without some or all of these specific details. In other instances, well known elements have not been described in detail in order not to unnecessarily obscure the present invention.

In preferred embodiments of the invention, the mobile POCT unit further comprises computing means for transmitting an identity of the mobile POCT unit for the central POCT manager obtaining location of the mobile POCT unit through a plurality of wireless identification readers connected to the central POCT manager. The reading of the transmitted identity of the mobile POCT unit through said wireless identification readers permits to the central POCT manager obtaining the position of the mobile POCT unit by applying triangulation techniques, according to the essentials of, for example, Multilateration processes for locating mobile phones.

Preferably, the computing means for wirelessly identifying a set of POCT analyzers comprise a wireless tag reader for reading wireless tags comprised in the POCT analyzers, each one of said wireless tags uniquely identifying the POCT analyzer on which the wireless tag is comprised.

In some embodiments of the invention, the computing means for transmitting an identity of the mobile POCT unit comprise an active RFID tag.

In a preferred embodiment of the invention, the wireless tag reader is a RFID (Radio-frequency identification) reader and the wireless tags comprised in the POCT analyzers are RFID tags compatible with the RFID reader.

Other wireless identification technologies different from RFID may be used for transmitting the identity of the mobile POCT unit and for wirelessly identifying the set of POCT analyzers. Examples of alternative wireless identification technologies are, for example Bluetooth, Infrared, etc. Nevertheless, RFID has the best balance between cost and reliability in relation to said alternatives. Moreover, some of said alternative technologies, as for example infrared, require alignment between the tag comprised in the analyzer and the reader comprised in the mobile POCT unit, which represents a big limitation.

In some embodiments, the RFID tags comprised in the POCT analyzers are passive RFID tags. Alternatively to the passive RFID tags, the RFID tags comprised in the POCT analyzers are semi-passive RFID tags. Active RFID tags may produce excessive interferences between transmissions, so not active RFID tags are preferred. Semi-passive tags may improve the reliability of transmissions in comparison with the passive tags, whereas passive tags may offer acceptable reliability with a lower cost.

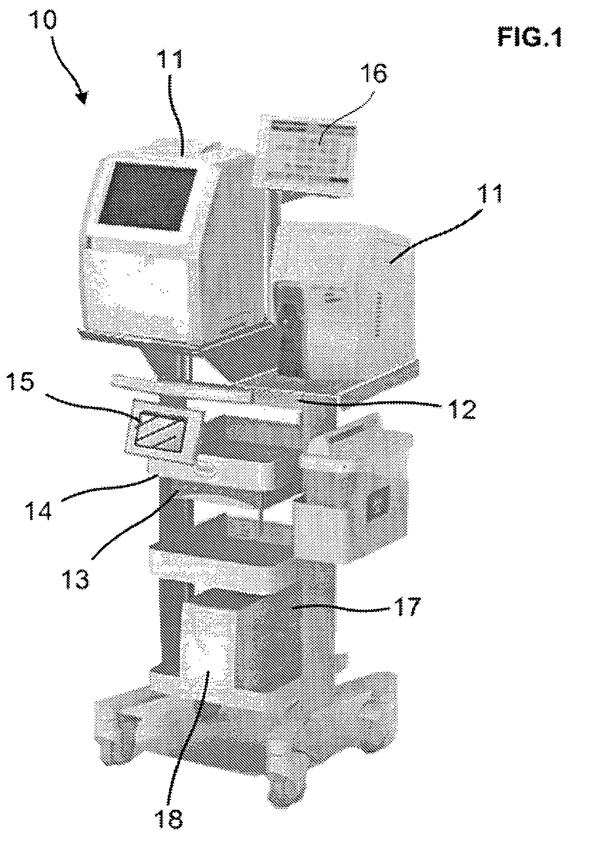

Figure 1 is a schematic representation of a mobile POCT unit with dynamic profile, according to an embodiment of the invention, said mobile POCT unit 10 comprising a plurality of dynamically linked analyzers 11, a computer 12, a WIFI router 13, an RFID scanner 14, an active RFID tag 15, a touch-screen 16, a battery 17 and an UPS (Uninterruptible Power Supply) 18.

The linked analyzers 11 may comprise the necessary functionalities for executing, for example, the following types of tests: glucometry, gasometry, urine analysis, cardiac reading, coagulation analysis, biochemical analysis, metabolite analysis and ion analysis, said types of tests or analytical parameters defining the current profile of the POCT unit, even though said profile may be dynamically changed by linking/unlinking different POCT analyzers.

Said analyzers 11 may be connected to the computer 12 through, for example, a docking station, the computer 12 executing the corresponding software for managing the analyzers, and the rest of devices comprised in the mobile POCT unit. Said software may comprise drivers and facilities related to the different analyzers 11 and other devices of the POCT unit. The computer 12 may also comprise a local repository of data for temporally storing data generated by the linked analyzers, said data to be finally sent to the central POCT manager.

The linked analyzers 11 may be operated without being connected to the computer 12 through the docking station, in which case the analytical data generated during said operation may be stored in the own memory of the analyzers. These analyzers, when connected to the docking station, may transfer all the accumulated data to the computer 12, so that the computer 12 may send, through the WIFI router 13, said data to the central POCT manager and other systems, for example: HIS (Hospital Information System) or LIS (Laboratory Information System), which are integrated information systems designed to manage the administrative, financial and clinical aspects of a Hospital (HIS) or of a Laboratory (LIS), or any other information system.

The RFID scanner 14 may be in charge of wirelessly identifying POCT analyzers, obtaining identification of the operators, obtaining identification of patients, etc. The RFID scanner 14 may be a radio-frequency identification system having the purpose of identification and tracking using radio waves. Said RFID device 14 may comprise two main parts, one is an integrated circuit for storing and processing information, modulating and demodulating a radio-frequency (RF) signal, and the second one is an antenna for receiving and transmitting the signal.

The WIFI router 13 may be in charge of managing the interconnection of the computer 12 comprised in the mobile POCT unit to the corresponding networks, allowing the routing of packets of data between the involved networks or determining the route that must take the packets of data. The computer 12 may send/receive data to/from the central POCT manager and to/from other systems, as for example HIS/LIS.

The active RFID tag 15 may be in charge of periodically transmitting an identity of the mobile POCT unit for the central POCT manager obtaining location of the mobile POCT unit through a plurality of wireless identification readers connected to the central POCT manager.

The touch screen 16 may be the main input/output device comprised in the mobile POCT unit and it allows to the operator to interact with all the devices of the system, that is to say, analyzers, computer, etc. Said touch screen 16 may display all or some of the received data, as for example: dynamic profile of the mobile POCT unit, a map representing a network of mobile POCT units with the position and profile of each mobile POCT unit, etc. In general, the touch screen 16 may be in charge of displaying any data related to any embodiment of the method for obtaining a dynamic profile of a mobile POCT unit from a central POCT manager.

The battery 17 and the UPS 18 are the power supply means associated to the electronic devices comprised in the mobile POCT unit. The battery may be rechargeable and has a high autonomy level, whereas the UPS is intended to maintain the power supply in case of bad operation of the battery. These power supply means avoid the use of wires for connecting the system to the common mains.

The mobile POCT unit may also comprise a tablet PC connected to the computer 12. The tablet PC may be equipped with integrated pen support, touch-screen support, digital-ink input, handwriting recognition technologies, RFID and Barcode Scanner (for patient and user recognition), Web-Camera, etc.

The computer 12 may also execute the computer program product comprising program instructions for causing a computer to perform any embodiment of the method for obtaining a dynamic profile of a mobile POCT unit from a central POCT manager.

In preferred embodiments of the invention, the central POCT manager further comprises computing means for obtaining location of the mobile POCT unit through a plurality of wireless identification readers connected to the central POCT manager, when the mobile POCT unit transmitting an identity of the mobile POCT unit. As commented before, the location of the mobile POCT unit may be obtained by applying triangulation techniques.

The central POCT manager may also comprise a repository for storing data related to each tagged POCT analyzer and related identities, dynamic profiles of mobile POCT units, status of each POCT unit, authorized operators, corrective quality controls, preventive quality controls and their frequency, identities of POCT units, acceptance quality and frequency ranges, predefined absence time threshold, etc.

The central POCT manager may also comprise a screen for displaying any data related to any embodiment of the method for providing a dynamic profile of a mobile POCT unit.

The central POCT manager may also comprise a computer for executing the computer program product comprising program instructions for causing a computer to perform any embodiment of the method for providing a dynamic profile of a mobile POCT unit.

In some embodiments of the invention, a network of mobile POCT units with dynamic profile comprises a central POCT manager system; at least one mobile POCT unit; and at least one POCT analyzer comprising wireless identification means for the mobile POCT unit wirelessly identifying said POCT analyzer.

Preferably, the network of mobile POCT units with dynamic profile further comprises a plurality of wireless identification readers connected to the central POCT manager for the central POCT manager obtaining location of the mobile POCT unit through said wireless identification readers connected to the central POCT manager, when the mobile POCT unit transmitting an identity of the mobile POCT unit.

A network of mobile POCT units may be understood as a network of mini-networks of analyzers, wherein each mini-network (or mobile POCT unit) may have a determined number of linked analyzers, so that only the operator of the POCT unit can operate with any of the linked analyzers. Thus, any analyzer is under strict control of the central POCT manager through the POCT unit to which the analyzer is linked.

Said configuration previously referred as a network of mini-networks of analyzers has also the advantage of decreasing interferences of wireless signals in comparison with a wireless network of analyzers (without intermediate mini-networks). Each mini-network interacts locally with the surrounding analyzers within a predetermined range, which may be reduced as required, so wireless signals between a mini-network and the surrounding analyzers may be short-range signals, whereas in a network without intermediate mini-networks the wireless signals for identifying analyzers have to be signals of longer range, said signals of longer range highly increasing the possibilities of interferences in relation to the short-range signals.

In such a network of mobile POCT units, the central POCT manager may comprise computing means for comparing different analytical results of identical samples from different mobile POCT units, so that said results may be correlated in real-time with the objective of estimating deviations in relation to predefined values of reference. Said deviations may be a good indicator of the reliability of the implied mobile POCT units.

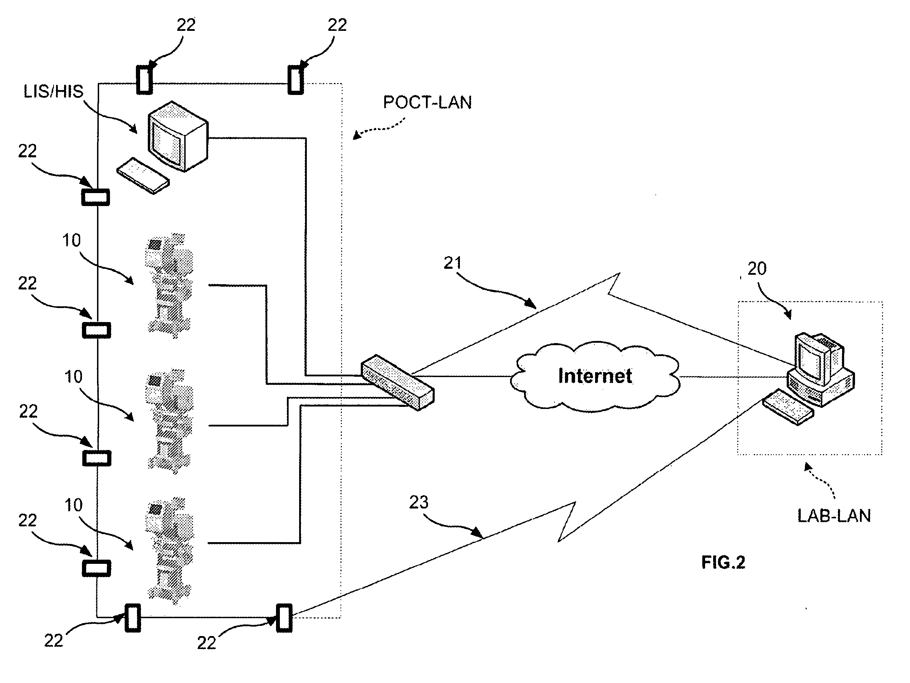

The figure 2 illustrates a preferred embodiment of a network of mobile POCT units, which comprises a plurality of mobile POCT units 10 and a central POCT manager system 20, all of said systems 10, 20 being connected by means of at least one communications network. In some preferred embodiments of the invention, the mobile POCT units comprised in a determined medical centre are connected through a specific LAN (POCT-LAN) and the central POCT manager 21 may be connected to a remote LAN (LAB-LAN), both networks POCT-LAN and LAB-LAN being connected through other intermediary networks, for example Internet. Even though, the POCT-LAN and the LAB-LAN may be connected through a point-to-point connection 21 due to, for example, speed reasons.

As the central laboratory, that is the entity monitoring the central POCT manager 20, can provide service to different medical centres, the network of mobile POCT units can comprise various POCT-LANs. Moreover, the Laboratory Information System and/or the Hospital Information System (LIS/HIS) can be connected to other networks. As introduced before, in case of existing diverse networks in the network of mobile POCT units, said diverse networks would be connected by means of intermediary networks, for example Internet.

More specifically, the figure 2 illustrates a preferred embodiment comprising one network POCT-LAN, to which the mobile POCT units are connected, one network LAB-LAN, to which the central POCT manager is connected, and Internet as the network connecting POCT-LAN and LAB-LAN.

Furthermore, the network of mobile POCT units may also comprises a plurality of wireless identification readers 22 connected 23 to the central POCT manager 20 for the central POCT manager 20 obtaining location of the mobile POCT unit 10 through said wireless identification readers 22 connected to the central POCT manager, when the mobile POCT unit 10 transmitting an identity of the mobile POCT unit.

In the method for obtaining a dynamic profile of a mobile POCT unit from a central POCT manager, the received dynamic profile may also comprise partial linked status which means that the related identified analyzer has related quality data partially satisfying quality requirements. For example, a POCT analyzer with two analytical parameters may satisfy the quality requirements related to one of said two analytical parameters, but may not satisfy the quality requirements related to the other analytical parameter, in which case said POCT analyzer may be partially linked in the sense of being only operative for processing the parameter having quality data satisfying the quality requirements.

In some embodiments of the invention, unlinked status may cause locking of the related POCT analyzer, so that the POCT analyzer will not be operative for any POCT unit and/or operator. In other embodiments, unlinked status may only cause marking the analyzer as not reliable without locking it. In this second case, the results obtained from said not reliable analyzer will be considered not validated by the central laboratory, so the operator of the unlinked (not reliable) analyzer will be the only responsible of the consequences.

In a preferred embodiment of the invention, the method for obtaining a dynamic profile of a mobile POCT unit from a central POCT manager further comprises:
- receiving, from the central POCT manager, a set of pre-linked status of an identified POCT analyzer, each pre-linked status representing that the identified POCT analyzer is a candidate to be operative for the mobile POCT unit;
- obtaining confirmation/cancellation of each pre-linked status;
- sending, to the central POCT manager, a set of confirmed pre-linked status comprising each confirmation of a pre-linked status.

In the method for obtaining a dynamic profile of a mobile POCT unit from a central POCT manager, the pre-linked status may also be partial pre-linked status according to the rule previously described in reference to the partial linked status.

In some embodiments, the method for obtaining a dynamic profile of a mobile POCT unit from a central POCT manager further comprises:
- obtaining an identity of an operator of the mobile POCT unit;
- sending said identity of the operator to the central POCT manager.

In preferred embodiments, the method for obtaining a dynamic profile of a mobile POCT unit from a central POCT manager further comprises:
- receiving, from the central POCT manager, a set of correctable unlinked status of an identified POCT analyzer, each correctable unlinked status comprising at least one corrective quality control to be executed on the identified POCT analyzer to correct the unlinked status;
- detecting, for each correctable unlinked status, completion/cancellation of its related at least one corrective quality control;
- sending, to the central POCT manager, a set of correctable unlinked status with completed corrective quality control, each correctable unlinked status with completed corrective quality control comprising the result of its related at least one corrective quality control.

In preferred embodiments of the invention, the method for obtaining a dynamic profile of a mobile POCT unit from a central POCT manager further comprises:
- receiving, from the central POCT manager, a set of periodic preventive quality controls, each periodic preventive quality control to be executed on an identified POCT analyzer with linked status;
- detecting completion/cancellation of each periodic preventive quality control;
- sending, to the central POCT manager, a set of completed periodic preventive quality controls, each completed periodic preventive quality control comprising the result of the completed periodic preventive quality control on its related identified POCT analyzer with linked status.

In some embodiments of the invention, the method for obtaining a dynamic profile of a mobile POCT unit from a central POCT manager further comprises transmitting an identity of the mobile POCT unit for the central POCT manager obtaining location of the mobile POCT unit through a plurality of wireless identification readers connected to the central POCT manager; and the received dynamic profile of the mobile POCT unit also comprises the location of the mobile POCT unit.

Preferably, the mobile POCT unit is comprised in a network of mobile POCT units with dynamic profile, and the method further comprises receiving, from the central POCT manager, dynamic profile of each mobile POCT unit of the network not being the mobile POCT unit.

A particular mobile POCT unit receiving dynamic profile of each POCT unit comprised in the network of mobile POCT units allows to the operator of the mobile POCT unit to decide extending the profile of the POCT unit by linking new analyzers or selecting an available new POCT unit in the network having the required profile. In case of the received dynamic profiles also including the position of its related mobile POCT unit, the operator may quickly locate and take the new POCT unit, having the required profile, instead of the current POCT unit, not having the required profile. This multi dynamic profile approach further increases the flexibility of the method.

In a preferred embodiment of the invention, the method for obtaining a dynamic profile of a mobile POCT unit from a central POCT manager further comprises:
- continuously repeating according to a predefined frequency:
   - wirelessly identifying a set of present POCT analyzers;
   - sending, to the central POCT manager, a present POCT analyzer identity for each identified present POCT analyzer.

This set of present POCT analyzers sent to the central POCT manager is useful for the central POCT manager obtaining linked but absent POCT analyzers, so that all of these linked but absent POCT analyzers having an accumulated time of absence exceeding a predefined absence time threshold may be automatically unlinked.

This absence time approach means that when an analyzer is out of the scope of the mobile POCT unit to which the analyzer is linked during a time exceeding a predefined absence time threshold, said absent analyzer is unlinked from the mobile POCT unit.

Apart from said unlinking modality based on absence time, the method for obtaining a dynamic profile of a mobile POCT unit from a central POCT manager may further comprise: obtaining a request for unlinking an identified POCT analyzer, for example from an operator; and sending said request to the central POCT manager, for the POCT manager determining unlinked status for the POCT analyzer related to the unlinking request.

In the method for obtaining a dynamic profile of a mobile POCT unit from a central POCT manager:
- receiving from the central POCT manager the dynamic profile of the mobile POCT unit
   may be executed periodically and/or each time the central POCT manager changes the status of some identified POCT analyzer.

In the embodiments of the method for obtaining a dynamic profile of a mobile POCT unit from a central POCT manager in which the mobile POCT unit is comprised in a network of mobile POCT units with dynamic profile:
- receiving from the central POCT manager dynamic profile of each mobile POCT unit of the network not being the mobile POCT unit
   may be executed periodically and/or each time the central POCT manager changes the status of some identified POCT analyzer.

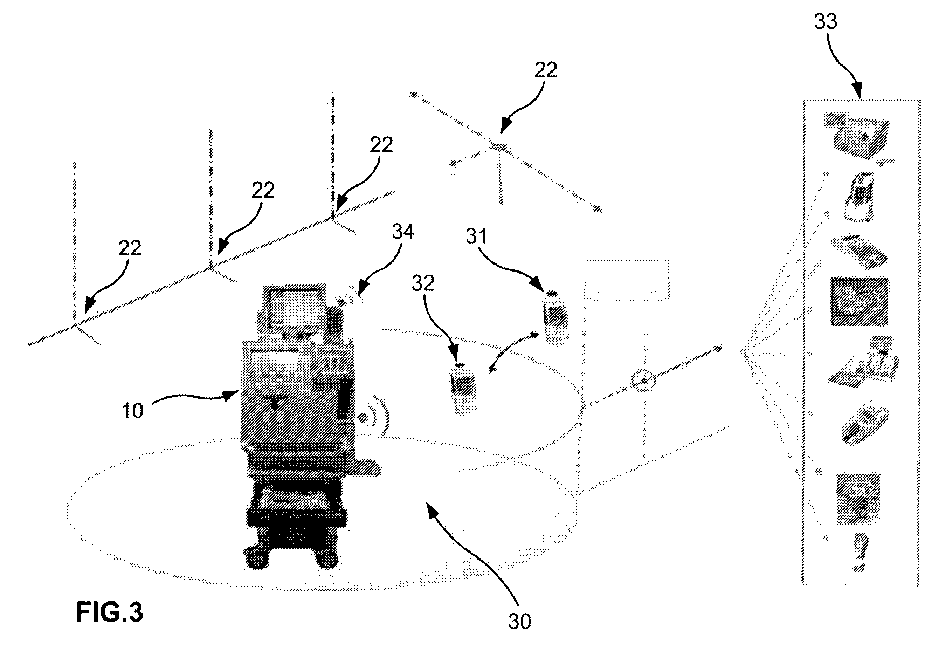

Figure 3 is a schematic representation of a mobile POCT unit and its related area of influence, according to an embodiment of the invention. This figure shows a mobile POCT unit 10 and its zone of influence 30 in which any analyzer, as for example the analyzer 32, may be wirelessly identified. The analyzer 31, for example, may not be identified because is out of the area of influence. Any other analyzer 33 may be identified, and linked if satisfying quality parameters, if placed within the area of influence.

The figure 3 also shows an active RFID tag 34 transmitting identity of the mobile POCT unit 10 for the central POCT manager obtaining location of the mobile POCT unit 10 through a plurality of wireless identification readers 22 connected to the central POCT manager.

In the method for providing a dynamic profile of a mobile POCT unit, the sent dynamic profile may also comprise partial linked status which means that the related identified analyzer has related quality data partially satisfying quality requirements. For example, a POCT analyzer with two analytical parameters may satisfy the quality requirements related to one of said two analytical parameters, but may not satisfy the quality requirements related to the other analytical parameter, in which case said POCT analyzer may be partially linked in the sense of being only operative for processing the parameter having quality data satisfying the quality requirements.

In some embodiments of the method for providing a dynamic profile of a mobile POCT unit, determining a linked status for each identified POCT analyzer having related quality data satisfying predefined quality requirements comprises:
- determining a pre-linked status for each identified POCT analyzer having related quality data satisfying quality requirements, said pre-linked status representing that the identified POCT analyzer is a candidate to be operative for the mobile POCT unit;
- sending, to the mobile POCT unit, a set of pre-linked status comprising each determined pre-linked status of an identified POCT analyzer;
- receiving, from the mobile POCT unit, a set of confirmed pre-linked status;
- determining a linked status for each identified POCT analyzer with confirmed pre-linked status, said linked status representing that the identified POCT analyzer is operative for the mobile POCT unit.

In the method for providing a dynamic profile of a mobile POCT unit, the pre-linked status may also be partial pre-linked status according to the rule previously described in reference to the partial linked status.

This approach of the method for providing a dynamic profile of a mobile POCT unit based on pre-linked status allows to the user select or confirm determined identified POCT analyzers to be linked to the mobile POCT unit. Then, not all the identified POCT analyzers satisfying quality parameters are linked to the POCT unit, but only the identified POCT analyzers satisfying quality parameters that are confirmed by the operator of the POCT unit.

In preferred embodiments of the method for providing a dynamic profile of a mobile POCT unit, related quality data comprises results of previously executed quality controls on its related identified POCT analyzer, and quality requirements comprise said results of previously executed quality controls being within acceptance quality and frequency range.

In preferred embodiments of the invention, the method for providing a dynamic profile of a mobile POCT unit further comprises receiving, from the mobile POCT unit, an identity of an operator of the mobile POCT unit.

In some embodiments of the method for providing a dynamic profile of a mobile POCT unit, related quality data comprises the identity of the operator of the mobile POCT unit, and quality requirements comprise said identity of the operator corresponding to an authorized operator profile.

Preferably, in the method for providing a dynamic profile of a mobile POCT unit, related quality data comprises the status of its related identified POCT analyzer, and quality requirements comprise said status not being linked to another mobile POCT unit different from the mobile POCT unit. In other words, a POCT analyzer may not be linked to more than one POCT unit at the same time.

In a preferred embodiment of the invention, the method for providing a dynamic profile of a mobile POCT unit further comprises:
- verifying, for each obtained unlinked status of an identified POCT analyzer, if execution of at least one corrective quality control on the identified POCT analyzer is required for correcting the unlinked status;
- sending, to the mobile POCT unit, a set of correctable unlinked status, each correctable unlinked status comprising its related at least one corrective quality control to be executed on its related identified POCT analyzer;
- receiving, from the mobile POCT unit, a set of correctable unlinked status with completed corrective quality control, each correctable unlinked status with completed corrective quality control comprising the result of its related at least one corrective quality control;
- determining a linked status for each identified POCT analyzer with correctable unlinked status with completed corrective quality control and having related quality data satisfying predefined quality requirements, said related quality data comprising the result of the related at least one completed corrective quality control, and said linked status representing that the identified POCT analyzer is operative for the mobile POCT unit.

This approach based on correctable unlinked status allows to the operator of the mobile POCT unit to convert an unlinked status into a linked status by executing determined quality controls required by the central POCT manager. Then, the flexibility of the method is further increased.

In some embodiments, the method for providing a dynamic profile of a mobile POCT unit further comprises:
- sending, to the mobile POCT unit, a set of periodic preventive quality controls, each periodic preventive quality control to be executed on an identified POCT analyzer with linked status;
- receiving, from the mobile POCT unit, a set of completed periodic preventive quality controls, each completed periodic preventive quality control comprising the result of the completed periodic preventive quality control on its related identified POCT analyzer with linked status;
- determining linked status for each identified POCT analyzer with completed periodic preventive quality control and having related quality data satisfying predefined quality requirements, said related quality data comprising the result of the related at least one periodic preventive quality control, and said linked status representing that the identified POCT analyzer is operative for the mobile POCT unit.

The central laboratory may define, for each POCT analyzer or for each different type or category of analyzers, preventive quality controls for ensuring reliability of the POCT analyzers, said preventive quality controls to be executed within a predetermined frequency, so that unsuccessful results of some of said preventive quality controls may cause unlinking of the "failed" POCT analyzer from the mobile POCT unit.

In preferred embodiments, the method for providing a dynamic profile of a mobile POCT unit further comprises:
- receiving, from the mobile POCT unit, an identity of the mobile POCT unit through a plurality of wireless identification readers connected to the central POCT manager;
- obtaining location of the mobile POCT unit from the identity of the mobile POCT unit received through said plurality of wireless identification readers; and the sent dynamic profile of the mobile POCT unit also comprises the location of the mobile POCT unit.

In preferred embodiments of the method for providing a dynamic profile of a mobile POCT unit, the mobile POCT unit is comprised in a network of mobile POCT units with dynamic profile; and the method further comprises:
- sending, to the mobile POCT unit, a dynamic profile of each mobile POCT unit of the network not being the mobile POCT unit.

In some embodiments of the invention, the method for providing a dynamic profile of a mobile POCT unit further comprises:
- when receiving from the mobile POCT unit a set of present POCT analyzer identities:
   - verifying, for each identified POCT analyzer with linked status, if the identified POCT analyzer with linked status is not a present POCT analyzer, said verification producing a set of linked but absent POCT analyzers;
   - obtaining accumulated absence time for each linked but absent POCT analyzer;
   - determining an unlinked status for each linked but absent POCT analyzer with accumulated absence time exceeding a predefined absence time threshold.

This absence time approach means that when an analyzer is out of the scope of the mobile POCT unit to which the analyzer is linked during a time exceeding a predefined absence time threshold, said absent analyzer is unlinked from the mobile POCT unit.

Apart from said unlinking modality based on absence time, the method for providing a dynamic profile of a mobile POCT unit may further comprise receiving, from the mobile POCT unit, a request for unlinking an identified POCT analyzer; and determining an unlinked status for said identified POCT analyzer.

In the method for providing a dynamic profile of a mobile POCT unit:
a sending to the mobile POCT unit the dynamic profile of the mobile POCT unit
   may be executed periodically and/or each time the central POCT manager changes the status of some identified POCT analyzer.

In the embodiments of the method for providing a dynamic profile of a mobile POCT unit in which the mobile POCT unit is comprised in a network of mobile POCT units with dynamic profile
- sending to the mobile POCT unit dynamic profile of each mobile POCT unit of the network not being the mobile POCT unit
   may be executed periodically and/or each time the central POCT manager changes the status of some identified POCT analyzer.

In a preferred embodiment of the invention, a method for determining a dynamic profile of at least one mobile POCT unit of a network of mobile POCT units comprises:
- the mobile POCT unit wirelessly identifying a set of POCT analyzers;
- the mobile POCT unit sending, to the central POCT manager, a POCT analyzer identity for each identified POCT analyzer;
- a central POCT manager receiving, from the mobile POCT unit, each POCT analyzer identity;
- the central POCT manager determining a linked status for each identified POCT analyzer having related quality data satisfying predefined quality requirements, said linked status representing that the identified POCT analyzer is operative for the mobile POCT unit;
- the central POCT manager determining unlinked status for each identified POCT analyzer having related quality data not satisfying predefined quality requirements, said unlinked status representing that the identified POCT analyzer is not operative for the mobile POCT unit;
- the central POCT manager sending, to the mobile POCT unit, the dynamic profile of the mobile POCT unit comprising the linked/unlinked status of each identified POCT analyzer;
- the mobile POCT unit receiving, from the central POCT manager, the dynamic profile of the mobile POCT unit comprising the linked/unlinked status of each identified POCT analyzer.

In some embodiments of the method for determining a dynamic profile of at least one mobile POCT unit of a network of mobile POCT units, the central POCT manager determining a linked status for each identified POCT analyzer having related quality data satisfying predefined quality requirements comprises:
- the central POCT manager determining a pre-linked status for each identified POCT analyzer having related quality data satisfying quality requirements, said pre-linked status representing that the identified POCT analyzer is a candidate to be operative for the mobile POCT unit;
- the central POCT manager sending, to the mobile POCT unit, each determined pre-linked status of an identified POCT analyzer; the method further comprises:
- the mobile POCT unit receiving, from the central POCT manager, each determined pre-linked status of an identified POCT analyzer;
- the mobile POCT unit obtaining confirmation/cancellation of each pre-linked status;
- the mobile POCT unit sending, to the central POCT manager, each confirmation of a pre-linked status;
and the central POCT manager determining a linked status for each identified POCT analyzer having related quality data satisfying predefined quality requirements further comprises:
• the central POCT manager receiving, from the mobile POCT unit, each confirmation of a pre-linked status;
• the central POCT manager determining a linked status for each identified POCT analyzer with confirmed pre-linked status, said linked status representing that the identified POCT analyzer is operative for the mobile POCT unit.

In preferred embodiments of the method for determining a dynamic profile of at least one mobile POCT unit of a network of mobile POCT units, related quality data comprises results of previously executed quality controls on its related identified POCT analyzer, and quality requirements comprise said results of previously executed quality controls being within acceptance quality and frequency range.

In preferred embodiments of the invention, the method for determining a dynamic profile of at least one mobile POCT unit of a network of mobile POCT units further comprises:
- the mobile POCT unit obtaining an identity of an operator of the mobile POCT unit;
- the mobile POCT unit sending the identity of the operator to the central POCT manager;
- the central POCT manager receiving, from the mobile POCT unit, the identity of the operator of the mobile POCT unit.

In some embodiments of the method for determining a dynamic profile of at least one mobile POCT unit of a network of mobile POCT units, related quality data comprises the identity of the operator of the mobile POCT unit, and quality requirements comprise said identity of the operator corresponding to an authorized operator profile.

Preferably, the method for determining a dynamic profile of at least one mobile POCT unit of a network of mobile POCT units, related quality data comprises the status of its related identified POCT analyzer, and quality requirements comprise said status not being linked to another mobile POCT unit different from the mobile POCT unit.

In a preferred embodiment of the invention, the method for determining a dynamic profile of at least one mobile POCT unit of a network of mobile POCT units further comprises:
- the central POCT manager verifying, for each unlinked status of an identified POCT analyzer, if execution of at least one corrective quality control on the identified POCT analyzer is required for correcting the unlinked status;
- the central POCT manager sending, to the mobile POCT unit, a set of correctable unlinked status, each correctable unlinked status comprising its related at least one corrective quality control to be executed on its related identified POCT analyzer;
- the mobile POCT unit receiving, from the central POCT manager, the set of correctable unlinked status;
- the mobile POCT unit detecting, for each correctable unlinked status, completion/cancellation of its related at least one corrective quality control;
- the mobile POCT unit sending, to the central POCT manager, each correctable unlinked status with completed corrective quality control comprising the result of its related at least one corrective quality control on its related identified POCT analyzer;
- the central POCT manager receiving, from the mobile POCT unit, each correctable unlinked status with completed corrective quality control comprising the result of its related at least one corrective quality control on its related identified POCT analyzer;
- the central POCT manager determining a linked status for each identified POCT analyzer with correctable unlinked status with completed corrective quality control and having related quality data satisfying predefined quality requirements, said related quality data comprising the result of the related at least one completed corrective quality control, and said linked status representing that the identified POCT analyzer is operative for the mobile POCT unit.

In some embodiments, the method for determining a dynamic profile of at least one mobile POCT unit of a network of mobile POCT units further comprises:
- the central POCT manager sending, to the mobile POCT unit, a set of periodic preventive quality controls, each periodic preventive quality control to be executed on an identified POCT analyzer with linked status;
- the mobile POCT unit receiving, from the central POCT manager, each periodic preventive quality control to be executed on an identified POCT analyzer with linked status;
- the mobile POCT unit detecting completion/cancellation of each periodic preventive quality control;
- the mobile POCT unit sending, to the central POCT manager, each completed periodic preventive quality control comprising the result of the completed periodic preventive quality control on its related identified POCT analyzer with linked status;
- the central POCT manager receiving, from the mobile POCT unit, each completed periodic preventive quality control comprising the result of the completed periodic preventive quality control on its related identified POCT analyzer with linked status;
- the central POCT manager determining a linked status for each identified POCT analyzer with completed periodic preventive quality control and having related quality data satisfying predefined quality requirements, said related quality data comprising the result of its related at least one periodic preventive quality control, and said linked status representing that the identified POCT analyzer is operative for the mobile POCT unit.

In preferred embodiments, the method for determining a dynamic profile of at least one mobile POCT unit of a network of mobile POCT units further comprises:
- the mobile POCT unit transmitting an identity of the mobile POCT unit for the central POCT manager obtaining location of the mobile POCT unit through a plurality of wireless identification readers connected to the central POCT manager;
- the central POCT manager receiving, from the mobile POCT unit, the identity of the mobile POCT unit through said plurality of wireless identification readers connected to the central POCT manager;
- the central POCT manager obtaining location of the mobile POCT unit from the identity of the mobile POCT unit received through said plurality of wireless identification readers;
and the dynamic profile of the mobile POCT unit also comprises the location of the mobile POCT unit.

In preferred embodiments of the method for determining a dynamic profile of at least one mobile POCT unit of a network of mobile POCT units, the mobile POCT unit is comprised in a network of mobile POCT units with dynamic profile; and the method further comprises:
- the central POCT manager sending, to the mobile POCT unit, a dynamic profile of each mobile POCT unit of the network not being the mobile POCT unit;
- the mobile POCT unit receiving, from the central POCT manager, the dynamic profile of each mobile POCT unit of the network not being the mobile POCT unit.

In some embodiments of the invention, the method for determining a dynamic profile of at least one mobile POCT unit of a network of mobile POCT units further comprises:
- the mobile POCT unit continuously repeating according to a predefined frequency:
   - the mobile POCT unit wirelessly identifying a set of present POCT analyzers;
   - the mobile POCT unit sending, to the central POCT manager, a present POCT analyzer identity for each identified present POCT analyzer;
- when the central POCT manager receiving from the mobile POCT unit a set of present POCT analyzer identities:
   - the central POCT manager verifying, for each identified POCT analyzer with linked status, if the identified POCT analyzer with linked status is not a present POCT analyzer, said verification producing a set of linked but absent POCT analyzers;
   - the central POCT manager obtaining accumulated absence time for each linked but absent POCT analyzer;
   - the central POCT manager determining an unlinked status for each linked but absent POCT analyzer with accumulated absence time exceeding a predefined absence time threshold.

Although this invention has been disclosed in the context of certain preferred embodiments and examples, it will be understood by those skilled in the art that the present invention extends beyond the specifically disclosed embodiments to other alternative embodiments and/or uses of the invention and obvious modifications and equivalents thereof. Thus, it is intended that the scope of the present invention herein disclosed should not be limited by the particular disclosed embodiments described before, but should be determined only by a fair reading of the claims that follow.

Further, although the embodiments of the invention described with reference to the drawings comprise computer apparatus and processes performed in computer apparatus, the invention also extends to computer programs, particularly computer programs on or in a carrier, adapted for putting the invention into practice. The program may be in the form of source code, object code, a code intermediate source and object code such as in partially compiled form, or in any other form suitable for use in the implementation of the processes according to the invention. The carrier may be any entity or device capable of carrying the program.

For example, the carrier may comprise a storage medium, such as a ROM, for example a CD ROM or a semiconductor ROM, or a magnetic recording medium, for example a floppy disc or hard disk. Further, the carrier may be a transmissible carrier such as an electrical or optical signal, which may be conveyed via electrical or optical cable or by radio or other means.

When the program is embodied in a signal that may be conveyed directly by a cable or other device or means, the carrier may be constituted by such cable or other device or means.

Alternatively, the carrier may be an integrated circuit in which the program is embedded, the integrated circuit being adapted for performing, or for use in the performance of, the relevant processes.

## Claims

1. A computer-implemented method for obtaining a profile of a mobile point-of-care-testing (POCT) unit (10) from a central POCT manager system (20), comprising the mobile POCT unit (10) performing the following steps:
- wirelessly identifying a set of present POCT analyzers (11, 32);
- sending, to the central POCT manager system (20), a present POCT analyzer identity for each identified present POCT analyzer (11, 32);
- receiving, from the central POCT manager system (20), the profile of the mobile POCT unit (10),
**characterized by**
said profile being a dynamic profile,
said mobile POCT unit (10) continuously repeating said steps according to a predefined frequency,
said dynamic profile comprising a linked/unlinked status for each identified present POCT analyzer (11, 32),
said linked status having been determined by the central POCT manager system (20) for each identified present POCT analyzer (11, 32) having related quality data satisfying predefined quality requirements, and representing that the identified present POCT analyzer (11, 32) is operative for the mobile POCT unit (10), and
said unlinked status having been determined by the central POCT manager system (20) for each identified present POCT analyzer (11, 32) having related quality data not satisfying predefined quality requirements, and representing that the identified present POCT analyzer (11, 32) is not operative for the mobile POCT unit (10).

2. The computer-implemented method according to claim 1, further comprising:
• receiving, from the central POCT manager system (20), a set of correctable unlinked status of an identified POCT analyzer (11, 32), each correctable unlinked status comprising at least one corrective quality control executable to be executed on the identified POCT analyzer (11, 32) to correct the unlinked status;
• detecting, for each correctable unlinked status, completion cancellation of its related at least one corrective quality control;
• sending, to the central POCT manager system (20), a set of correctable unlinked status with completed corrective quality control, each correctable unlinked status with completed corrective quality control comprising the result of its related at least one executed corrective quality control.

3. The computer-implemented method according to any of claims 1 or 2, further comprising:
• receiving, from the central POCT manager system (20), a set of periodic preventive quality control executables, each periodic preventive quality control executable to be executed on an identified POCT analyzer (11, 32) with linked status;
• detecting completion/cancellation of each periodic preventive quality control;
• sending, to the central POCT manager system (20), a set of results of the completed periodic preventive quality controls, each completed periodic preventive quality control comprising the result of the completed periodic preventive quality control on its related identified POCT analyzer (11, 32) with linked status.

4. The computer-implemented method according to any of claims 1 to 3, further comprising:
• transmitting an identity of the mobile POCT unit (10) for the central POCT manager system (20) obtaining location of the mobile POCT unit (10) through a plurality of wireless identification readers (22) connected to the central POCT manager system (20);
and wherein the received dynamic profile of the mobile POCT unit (10) also comprises the location of the mobile POCT unit (10).

5. The computer-implemented method according to any of claims 1 to 4, wherein the mobile POCT unit (10) is a first mobile POCT unit (10) comprised in a network of mobile POCT units (10) with dynamic profile; and wherein the computer-implemented method further comprises:
- receiving, from the central POCT manager system (20), the dynamic profile of each mobile POCT unit (10) of the network not being the first mobile POCT unit (10).

6. The computer-implemented method according to any of claims 1 to 5, further comprising:
• continuously repeating according to a predefined frequency:
• wirelessly identifying a set of present POCT analyzers (11, 32);
• sending, to the central POCT manager system (20), a present POCT analyzer identity for each identified present POCT analyzer (11, 32).

7. A computer program product comprising program instructions for causing a computer to perform, when said program is run on said computer, a computer-implemented method for obtaining a dynamic profile of a mobile point of care testing (POCT) unit (10) from a central POCT manager system (20), said computer-implemented method according to any of claims 1 to 6.

8. A computer-implemented method of a central point-of-care-testing (POCT) manager system (20) providing a profile of a mobile POCT unit (10), comprising:
- receiving from the mobile POCT unit (10) a set of present POCT analyzer identities, said set of present POCT analyzer identities having been wirelessly identified by the mobile POCT unit (10);
- determining the profile of the mobile POCT unit (10);
- sending, to the mobile POCT unit (10), the profile of the mobile POCT unit (10),
**characterized in that**
said profile is a dynamic profile,
said mobile POCT unit (10) continuously repeats its steps according to a predefined frequency,
the dynamic profile comprises a linked/unlinked status of each identified present POCT analyzer (11, 32), and **in that** said determining the dynamic profile of the mobile POCT unit (10) comprises:
- determining the linked status for each identified present POCT analyzer (11, 32) having related quality data satisfying predefined quality requirements, said linked status representing that the identified present POCT analyzer (11, 32) is operative for the mobile POCT unit (10);
- determining the unlinked status for each identified present POCT analyzer (11, 32) having related quality data not satisfying predefined quality requirements, said unlinked status representing that the identified present POCT analyzer (11, 32) is not operative for the mobile POCT unit (10).

9. The computer-implemented method according to claim 8, wherein related quality data comprises results of previously executed quality control executables on its related identified POCT analyzer (11, 32), and quality requirements comprise said results of previously executed quality control executables being within acceptance quality and frequency range.

10. The computer-implemented method according to any of claims 8 or 9, wherein related quality data comprises the status of its related identified POCT analyzer (11, 32), and quality requirements comprise said status not being linked to another mobile POCT unit (10) different from the mobile POCT unit (10).

11. The computer-implemented method according to any of claims 8 to 10, further comprising:
• verifying, for each obtained unlinked status of an identified POCT analyzer (11, 32), if execution of at least one corrective quality control executable on the identified POCT analyzer (11, 32) is required for correcting the unlinked status;
• sending, to the mobile POCT unit (10), a set of correctable unlinked status, each correctable unlinked status comprising its related at least one corrective quality control executable to be executed on its related identified POCT analyzer (11, 32);
• receiving, from the mobile POCT unit (10), a set of correctable unlinked status with completed corrective quality control, each correctable unlinked status with completed corrective quality control comprising the result of its related at least one executed corrective quality control;
• determining a linked status for each identified POCT analyzer (11, 32) with correctable unlinked status with completed corrective quality control and having related quality data satisfying predefined quality requirements, said related quality data comprising the result of the related at least one completed corrective quality control, and said linked status representing that the identified POCT analyzer (11, 32) is operative for the mobile POCT unit (10).

12. The computer-implemented method according to any of claims 8 to 11, further comprising:
• sending, to the mobile POCT unit (10), a set of periodic preventive quality control executables, each periodic preventive quality control executable to be executed on an identified POCT analyzer (11, 32) with linked status;
• receiving, from the mobile POCT unit (10), a set of completed periodic preventive quality controls, each completed periodic preventive quality control comprising the result of the completed periodic preventive quality control on its related identified POCT analyzer (11, 32) with linked status;
• determining linked status for each identified POCT analyzer (11, 32) with completed periodic preventive quality control and having related quality data satisfying predefined quality requirements, said related quality data comprising the result of the related at least one executed periodic preventive quality control, and said linked status representing that the identified POCT analyzer (11, 32) is operative for the mobile POCT unit (10).

13. The computer-implemented method according to any of claims 8 to 12, further comprising:
• when receiving from the mobile POCT unit (10) a set of present POCT analyzer identities:
• verifying, for each identified POCT analyzer (11, 32) with linked status, if the identified POCT analyzer (11, 32) with linked status is not a present POCT analyzer (11, 32), said verification producing a set of linked but absent POCT analyzers (31);
• obtaining accumulated absence time for each linked but absent POCT analyzer (31);
• determining an unlinked status for each linked but absent POCT analyzer (31) with accumulated absence time exceeding a predefined absence time threshold.

14. A mobile point-of-care-testing (POCT) unit (10) with dynamic profile, comprising computing means adapted to carry out each of the steps of claim 1.

15. A central point-of-care-testing (POCT) manager system (20) comprising computing means adapted to carry out each of the steps of claim 8.

16. A network of mobile point-of-care-testing (POCT) units (10) with dynamic profile, comprising:
- a central POCT manager system (20) according to claim 15;
- at least one mobile POCT unit (10) according to claim 14;
- at least one POCT analyzer (11, 32) comprising wireless identification means for the mobile POCT unit (10) wirelessly identifying said POCT analyzer (11, 32).

## Patentansprüche

1. Ein computerimplementiertes Verfahren zum Erhalten eines Profils einer mobilen Point-of-Care-Testing (POCT)-Einheit (10) aus einem zentralen POCT-Managersystem (20), wobei die mobile POCT-Einheit (10) das Durchführen der folgenden Schritte umfasst:
- das drahtlose Identifizieren einer Reihe von anwesenden POCT-Analysatoren (11, 32);
- das Senden der Identität eines anwesenden POCT-Analysators zum zentralen POCT-Managersystem (20) für jeden identifizierten anwesenden POCT-Analysator (11, 32);
- das Empfangen des Profils der mobilen POCT-Einheit (10) aus dem zentralen POCT-Managersystem (20),
**dadurch gekennzeichnet, dass**
das Profil ein dynamisches Profil ist,
die mobile POCT-Einheit (10) die obigen Schritte gemäß einer vorherbestimmten Frequenz fortlaufend wiederholt,
das dynamische Profil einen verknüpften/nicht verknüpften Status für jeden identifizierten anwesenden POCT-Analysator (11, 32) umfasst,
wobei der verknüpfte Status für jeden identifizierten anwesenden POCT-Analysator (11, 32) mit zugehörigen Qualitätsdaten, die vorherbestimmte Qualitätsanforderungen erfüllen, durch das zentrale POCT-Managersystem (20) festgestellt worden ist und der verknüpfte Status zeigt, dass der identifizierte anwesende POCT-Analysator (11, 32) für die mobile POCT-Einheit (10) operativ ist, und
wobei der nicht verknüpfte Status für jeden identifizierten anwesenden POCT-Analysator (11, 32) mit zugehörigen Qualitätsdaten, die vorherbestimmte Qualitätsanforderungen nicht erfüllen durch das zentrale POCT-Managersystem (20) ermittelt worden ist und der nicht verknüpfter Status zeigt, dass der identifizierte anwesende POCT-Analysator (11, 32) für die mobile POCT-Einheit (10) nicht operativ ist.

2. Das computerimplementierte Verfahren nach Anspruch 1, weiterhin umfassend:
• das Empfangen von einer Reihe von korrigierbaren nicht verknüpften Status eines identifizierten POCT-Analysators (11, 32) aus dem zentralen POCT-Managersystem (20), wobei jeder korrigierbare nicht verknüpfte Status mindestens eine korrektive ausführbare Qualitätskontrolldatei umfasst, welche für den identifizierten POCT-Analysator (11, 32) auszuführen ist, um den nicht verknüpften Status zu korrigieren;
• das Ermitteln, für jeden korrigierbaren nicht verknüpften Status, von Beendigung/Abbruchvorgängen von seiner zugehörigen mindestens eine korrektive Qualitätskontrolle;
• das Senden von einer Reihe von korrigierbaren nicht verknüpften Status mit beendeter korrektiver Qualitätskontrolle zum zentralen POCT-Managersystem (20), wobei jeder korrigierbare nicht verknüpfte Status mit beendeter korrektiver Qualitätskontrolle das Ergebnis seiner zugehörigen mindestens eine durchgeführte korrektive Qualitätskontrolle umfasst.

3. Das computerimplementierte Verfahren nach einem der Ansprüche 1 bis 2 weiterhin umfassend:
• das Empfangen von einer Reihe von periodischen präventiven ausführbaren Qualitätskontrolldateien aus dem zentralen POCT-Managersystem (20), wobei jede periodische präventive ausführbare Qualitätskontrolldatei für einen identifizierten POCT-Analysator (11, 32) mit verknüpften Status auszuführen ist;
• das Ermitteln von Beendigung/Abbruchvorgängen jeder periodischen präventiven Qualitätskontrolle;
• das Senden von einer Reihe von Ergebnissen der beendeten periodischen präventiven Qualitätskontrollen zum zentralen POCT-Managersystem (20), wobei jede beendete periodische präventive Qualitätskontrolle das Ergebnis von der beendeten periodischen präventiven Qualitätskontrolle für deren zugehörigen identifizierten POCT-Analysator (11, 32) mit verknüpften Status umfasst.

4. Das computerimplementierte Verfahren nach einem der Ansprüche 1 bis 3, weiterhin umfassend:
• das Übertragen von einer Identität der mobilen POCT-Einheit (10) für das zentrale POCT-Managersystem (20), indem der Standort der mobilen POCT-Einheit (10) mittels einer Vielzahl von drahtlosen Identifikationslesern (22) erhalten wird, die mit dem zentralen POCT-Managersystem (20) verbunden sind; und wobei das empfangene dynamische Profil der mobilen POCT-Einheit (10) auch den Standort der mobilen POCT-Einheit (10) umfasst.

5. Das computerimplementierte Verfahren nach einem der Ansprüche 1 bis 4, wobei die mobile POCT-Einheit (10) eine erste mobile POCT-Einheit (10) ist, welche in einem Netzwerk von mobilen POCT-Einheiten (10) mit dynamischem Profil enthalten ist; und wobei das computerimplementierte Verfahren weiterhin folgendes umfasst:
- das Erhalten des dynamischen Profils jeder mobilen POCT-Einheit (10) des Netzwerks, die nicht die erste mobile POCT-Einheit (10) sind aus dem zentralen POCT-Managersystem (20).

6. Das computerimplementierte Verfahren nach einem der Ansprüche 1 bis 5 weiterhin umfassend:
• das fortlaufende Wiederholen der folgenden Schritte gemäß einer vorherbestimmten Frequenz:
• das drahtlose Identifizieren von einer Reihe von anwesenden POCT-Analysatoren (11, 32);
• das Senden, für jeden identifizierten anwesenden POCT-Analysator (11, 32), einer Identität des anwesenden POCT-Analysators zum zentralen POCT-Managersystem (20).

7. Ein Computerprogrammprodukt umfassend Programmanweisungen um herbeizuführen, dass ein Computer ein computerimplementiertes Verfahren zum Erhalten eines dynamischen Profils einer mobilen Point-of-Case-Testing (POCT)-Einheit (10) aus einem zentralen POCT-Managersystem (20) durchführt, wenn das Programm in dem Computer ausgeführt wird, wobei das computerimplementierte Verfahren nach einem der Ansprüche 1 bis 6 ist

8. Ein computerimplementiertes Verfahren eines zentralen Point-of-Care-Testing (POCT)-Managersystems (20), welches ein Profil einer mobilen POCT-Einheit (10) bereitstellt, umfassend:
- das Empfangen von einer Reihe von Identitäten von anwesenden POCT-Analysatoren aus der mobilen POCT-Einheit (10), wobei die Reihe von Identitäten von anwesenden POCT-Analysatoren durch die mobile POCT-Einheit (10) drahtlos identifiziert worden sind;
- das Feststellen des Profils der mobilen POCT-Einheit (10);
- das Senden des Profils der mobilen POCT-Einheit (10) zu der mobilen POCT-Einheit (10), **dadurch gekennzeichnet, dass**
das Profil ein dynamisches Profil ist,
die mobile POCT-Einheit (10) fortlaufend die Schritte gemäß einer vorherbestimmten Frequenz wiederholt,
das dynamische Profil einen verknüpften/nicht verknüpften Status von jedem identifizierten anwesenden POCT-Analysator (11, 32) umfasst, und dadurch, dass das Feststellen des dynamischen Profils der mobilen POCT-Einheit (10) folgendes umfasst:
- das Feststellen des verknüpften Status für jeden identifizierten anwesenden POCT-Analysator (11, 32) mit zugehörigen Qualitätsdaten, die vorherbestimmte Qualitätsanforderungen erfüllen, wobei der verknüpfte Status zeigt, dass der identifizierte anwesende POCT-Analysator (11, 32) für die mobile POCT-Einheit (10) operativ ist;
- das Feststellen des nicht verknüpften Status für jeden identifizierten anwesenden POCT-Analysator (11, 32) mit zugehörigen Qualitätsdaten, die vorherbestimmte Qualitätsanfoderungen nicht erfüllen, wobei der nicht verknüpfte Status zeigt, dass der identifizierte anwesende POCT-Analysator (11, 32) für die mobile POCT-Einheit (10) nicht operativ ist.

9. Das computerimplementierte Verfahren nach Anspruch 8, wobei die zugehörigen Qualitätsdaten Ergebnisse von vorher ausgeführten ausführbaren Qualitätskontrolldateien für den zugehörigen identifizierten POCT-Analysator (11, 32) umfassen, und die Qualitätsanforderungen die Ergebnisse von vorher ausgeführten ausführbaren Qualitätskontrolldateien umfassen, die in einem annehmbaren Qualitäts- und Frequenzbereich liegen.

10. Das computerimplementierte Verfahren nach einem der Ansprüche 8 oder 9, wobei die zugehörigen Qualitätsdaten jeweils den Status von den zugehörigen identifizierten POCT-Analysatoren (11, 32) umfassen, und die Qualitätsanforderungen den Status umfassen, der mit keiner anderen mobilen POCT-Einheit (10) als der mobilen POCT-Einheit (10) verknüpft ist.

11. Das computerimplementierte Verfahren nach einem der Ansprüche 8 bis 10, weiterhin umfassend:
• das Verifizieren, für jeden erhaltenen nicht verknüpften Status eines identifizierten POCT-Analysators (11, 32), ob die Ausführung von mindestens einer korrektiven ausführbaren Qualitätskontrolldatei für den identifizierten POCT-Analysator (11, 32) erforderlich ist, um den nicht verknüpften Status zu korrigieren;
• das Senden von einer Reihe von korrigierbaren nicht verknüpften Status zu der mobilen POCT-Einheit (10), wobei jeder korrigierbare nicht verknüpfte Status seine zugehörige mindestens eine korrektive ausführbare Qualitätskontrolldatei umfasst, die für ihren zugehörigen identifizierten POCT-Analysator (11, 32) auszuführen ist;
• das Empfangen von einer Reihe von korrigierbaren nicht verknüpften Status mit beendeter korrektiver Qualitätskontrolle aus der mobilen POCT-Einheit (10), wobei jeder korrigierbare nicht verknüpfte Status mit beendeter korrektiver Qualitätskontrolle das Ergebnis von seiner zugehörigen mindestens einen durchgeführten korrektiven Qualitätskontrolle umfasst;
• das Feststellen von einem verknüpften Status für jeden identifizierten POCT-Analysator (11, 32) mit korrigierbarem nicht verknüpftem Status mit beendeter korrektiver Qualitätskontrolle und mit zugehörigen Qualitätsdaten, die vorherbestimmte Qualitätsanforderungen erfüllen, wobei die zugehörigen Qualitätsdaten das Ergebnis von der zugehörigen mindestens einen beendeten korrektiven Qualitätskontrolle umfassen, und der verknüpfte Status zeigt, dass der identifizierte POCT-Analysator (11, 32) für die mobile POCT-Einheit (10) operativ ist.

12. Das computerimplementierte Verfahren nach einem der Ansprüche 8 bis 11 weiterhin umfassend:
• das Senden von einer Reihe von periodischen präventiven ausführbaren Qualitätskontrolldateien zu der mobilen POCT-Einheit (10), wobei jede periodische präventive ausführbare Qualitätskontrolldatei für einen identifizierten POCT Analysator (11, 32) mit verknüpftem Status auszuführen ist;
• das Empfangen von einer Reihe von beendeten periodischen präventiven Qualitätskontrollen aus der mobilen POCT-Einheit (10), wobei jede beendete periodische präventive Qualitätskontrolle das Ergebnis von der beendeten periodischen präventiven Qualitätskontrolle für ihren zugehörigen identifizierten POCT-Analysator (11, 32) mit verknüpftem Status umfasst;
• das Feststellen des verknüpften Status für jeden identifizierten POCT-Analysator (11, 32) mit beendeter periodischer präventiver Qualitätskontrolle und mit zugehörigen Qualitätsdaten, die vorherbestimmte Qualitätsanforderungen erfüllen, wobei die zugehörigen Qualitätsdaten das Ergebnis von derzugehörigen mindestens eine durchgeführte periodische präventive Qualitätskontrolle umfassen, und wobei der verknüpfte Status zeigt, dass der identifizierte POCT-Analysator (11, 32) für die mobile POCT-Einheit (10) operativ ist.

13. Das computerimplementierte Verfahren nach einem der Ansprüche 8 bis 12 weiterhin umfassend:
• wenn aus der mobilen POCT-Einheit (10) eine Reihe von Identitäten von anwesenden POCT-Analysatoren empfangen wird:
• das Verifizieren, für jeden identifizierten POCT-Analysator (11, 32) mit verknüpftem Status, ob der identifizierte POCT-Analysator (11, 32) mit verknüpftem Status nicht ein anwesender POCT-Analysator (11, 32) ist, wobei die Verifikation eine Reihe von verknüpften aber abwesenden POCT-Analysatoren (31) ergibt;
• das Erhalten von kumulierter Abwesenheitszeit für jeden verknüpften aber abwesenden POCT-Analysator (31);
• das Feststellen eines nicht verknüpften Status für jeden verknüpften aber abwesenden POCT-Analysator (31) mit kumulierter Abwesenheitszeit über einen vorherbestimmten Abwesenheitszeitschwellenwert hinaus.

14. Eine mobile Point of Care-Testing (POCT)-Einheit (10) mit dynamischem Profil, umfassend Computervorrichtungen, die angepasst sind, um jeden Schritt des Anspruchs 1 durchzuführen.

15. Ein zentrales Point-of-Care-Testing (POCT)-Managersystem (20) umfassend Computervorrichtungen, die angepasst sind, um jeden Schritt des Anspruchs 8 durchzuführen.

16. Ein Netzwerk von mobilen Point of Care-Testing (POCT)-Einheiten (10) mit dynamischem Profil, umfassend:
- ein zentrales POCT-Managersystem (20) nach Anspruch 15;
- mindestens eine mobile POCT-Einheit (10) nach Anspruch 14;
- mindestens einen POCT-Analysator (11, 32) umfassend ein Mittel zur drahtlosen Identifizierung für die mobile POCT-Einheit (10), die den POCT-Analysator (11, 32) drahtlos identifiziert.

## Revendications

1. Un procédé mis en oeuvre par ordinateur pour obtenir un profil d'une unité d'analyse au chevet du patient (POCT) mobile (10) à partir d'un système de gestion POCT central (20), comprenant l'unité POCT mobile (10) effectuer las étapes suivantes :
- identifier sans fil une série d'analyseurs POCT présents (11, 32);
- envoyer, au système de gestion POCT central (20), une identité d'analyseur POCT présent pour chaque analyseur POCT présent (11, 32) identifié ;
- recevoir, du système de gestion POCT central (20), le profil de l'unité POCT mobile (10),
**caractérisé en ce que**
ledit profil est un profil dynamique,
ladite unité POCT mobile (10) répète de façon continue lesdites étapes selon une fréquence prédéfinie,
ledit profil dynamique comprend un état lié/non lié pour chaque analyseur POCT présent (11, 32) identifié,
ayant été déterminé ledit état lié par le système de gestion POCT central (20) pour chaque analyseur POCT présent (11, 32) identifié ayant des données de qualité associées qui satisfont des exigences de qualité prédéfinies, et représentant que l'analyseur POCT présent (11, 32) identifié est opérationnel pour l'unité POCT mobile (10), et
ayant été déterminé ledit état non lié par le système de gestion POCT central (20) pour chaque analyseur POCT présent (11, 32) identifié ayant des données de qualité associées qui ne satisfont pas des exigences de qualité prédéfinies, et représentant que l'analyseur POCT présent (11, 32) identifié n'est pas opérationnel pour l'unité POCT mobile (10).

2. Procédé mis en oeuvre par ordinateur selon la revendication 1, comprenant en outre :
• recevoir, du système de gestion POCT central (20), une série d'états non liés corrigibles d'un analyseur POCT (11, 32) identifié, comprenant chaque état non lié corrigible au moins un exécutable de contrôle de qualité correctif devant être exécuté pour l'analyseur POCT (11, 32) identifié afin de corriger l'état non lié ;
• détecter, pour chaque état non lié corrigible, achèvement / interruption de son au moins un contrôle de qualité correctif associé ;
• envoyer, au système de gestion POCT central (20), une série d'états non liés corrigibles avec contrôle de qualité correctif achevé, comprenant chaque état non lié corrigible avec contrôle de qualité correctif achevé le résultat de son au moins un contrôle de qualité correctif exécuté associé.

3. Le procédé mis en oeuvre par ordinateur selon l'une quelconque des revendications 1 à 2, comprenant en outre :
• recevoir du système de gestion POCT central (20), une série d'exécutables de contrôle de qualité préventif périodique, devant être exécuté chaque exécutable de contrôle de qualité préventif périodique pour un analyseur POCT (11, 32) identifié ayant un état lié ;
• détection d'achèvement / interruption de chaque contrôle de qualité préventif périodique ;
• envoyer, au système de gestion POCT central (20), une série de résultats des contrôles de qualité préventifs périodiques achevés, comprenant chaque contrôle de qualité préventif périodique achevé le résultat du contrôle de qualité préventif périodique achevé pour son analyseur POCT (11, 32) identifié associé avec un état lié.

4. Le procédé mis en oeuvre par ordinateur selon l'une quelconque des revendications 1 à 3, comprenant en outre :
• transmettre une identité de l'unité POCT mobile (10) pour le système de gestion POCT central (20) obtenant la position de l'unité POCT mobile (10) moyennant une pluralité de lecteurs d'identification sans fils (22) reliés au système de gestion POCT central (20) ;
et dans lequel le profil dynamique reçu de l'unité POCT mobile (10) comprend en outre la position de l'unité POCT mobile (10).

5. Le procédé mis en oeuvre par ordinateur selon l'une quelconque des revendications 1 à 4, dans lequel l'unité POCT mobile (10) est une première unité POCT mobile (10) comprise dans un réseau d'unités POCT mobiles (10) avec profil dynamique ; et dans lequel le procédé mis en oeuvre par ordinateur comprend en outre :
- recevoir, du système de gestion POCT central (20), le profil dynamique de chaque unité POCT mobile (10) du réseau qui n'est pas la première unité POCT mobile (10).

6. Le procédé mis en oeuvre par ordinateur selon l'une quelconque des revendications 1 à 5, comprenant en outre :
• répéter de façon continue selon une fréquence prédéfinie :
• identifier sans fils une série d'analyseurs POCT présents (11, 32);
• envoyer, au système de gestion POCT central (20), une identité d'analyseur POCT présent pour chaque analyseur POCT présent (11, 32) identifié.

7. Un produit de programme d'ordinateur comprenant des instructions de programme pour entraîner l'exécution par un ordinateur, lorsque ledit programme est exécuté dans ledit ordinateur, d'un procédé mis en oeuvre par ordinateur pour obtenir un profil dynamique d'une unité d'analyse au chevet du patient (POCT) mobile (10) d'un système de gestion POCT central (20), étant ledit procédé mis en oeuvre par ordinateur selon l'une quelconque des revendications 1 à 6.

8. Un procédé mis en oeuvre par ordinateur d'un système de gestion central (20) d'analyse au chevet du patient (POCT) fournissant un profile d'une unité POCT mobile (10), comprenant :
- recevoir de l'unité POCT mobile (10) une série d'identités d'analyseurs POCT présents, ayant été identifiée ladite série d'identités d'analyseurs POCT présents sans fils par l'unité POCT mobile (10) ;
- déterminer le profil de l'unité POCT mobile (10) ;
- envoyer, à l'unité POCT mobile (10), le profil de l'unité POCT mobile (10),
**caractérisé en ce que**
ledit profil est un profil dynamique,
ladite unité POCT mobile (10) répète de façon continue ses étapes selon une fréquence prédéfinie,
le profil dynamique comprend un état lié/non lié de chaque analyseur POCT présent (11, 32) identifié, et **en ce que** ladite détermination du profil dynamique de l'unité POCT mobile (10) comprend :
- déterminer l'état lié pour chaque analyseur POCT présent (11, 32) identifié ayant des données de qualité associées satisfaisant des exigences de qualité prédéfinies, représentant ledit état lié que l'analyseur POCT présent (11, 32) identifié est opérationnel pour l'unité POCT mobile (10) ;
- déterminer l'état non lié pour chaque analyseur POCT présent (11, 32) identifié ayant des données de qualité associées qui ne satisfont pas des exigences de qualité prédéfinies, représentant ledit état non lié que l'analyseur POCT présent (11, 32) identifié n'est pas opérationnel pour l'unité POCT mobile (10).

9. Le procédé mis en oeuvre par ordinateur selon la revendication 8, dans lequel les données de qualité associées comprennent des résultats d'exécutables de contrôle de qualité antérieurement exécutés pour leur analyseur POCT (11, 32) identifié associé, et les exigences de qualité comprennent lesdits résultats d'exécutables de contrôle de qualité antérieurement exécutés qui sont dans un intervalle de qualité et de fréquence acceptable.

10. Le procédé mis en oeuvre par ordinateur selon l'une quelconque des revendications 8 ou 9, dans lequel les donnés de qualité associées comprennent l'état de leur analyseur POCT (11, 32) identifié associé, et les exigences de qualité comprennent ledit état non lié à une autre unité POCT mobile (10) différente de l'unité POCT mobile (10).

11. Le procédé mis en oeuvre par ordinateur selon l'une quelconque des revendications 8 à 10, comprenant en outre :
• vérifier, pour chaque état non lié obtenu d'un analyseur POCT (11,32) identifié, si l'exécution d'au moins un exécutable de contrôle de qualité correctif pour l'analyseur POCT (11, 32) identifié est nécessaire pour corriger l'état non lié ;
• envoyer, à l'unité POCT mobile (10), une série d'états non liés corrigibles, comprenant chaque état non lié corrigible son au moins un exécutable de contrôle de qualité correctif associé devant être exécuté pour son analyseur POCT (11, 32) identifié associé ;
• recevoir, de l'unité POCT mobile (10), une série d'états non liés corrigibles avec contrôle de qualité corrigible achevé, comprenant chaque état non lié corrigible avec contrôle de qualité corrigible achevé le résultat de son au moins un contrôle de qualité correctif exécuté associé ;
• déterminer un état lié pour chaque analyseur POCT (11, 32) identifié avec un état non lié corrigible avec contrôle de qualité correctif achevé et ayant des données de qualité associées satisfaisant des exigences de qualité prédéfinies, comprenant lesdites données de qualité associées le résultat de l'au moins un contrôle de qualité correctif achevé associé, et représentant ledit état lié que l'analyseur POCT (11, 32) identifié est opérationnel pour l'unité POCT mobile (10).

12. Le procédé mis en oeuvre par ordinateur selon l'une quelconque des revendications 8 à 11, comprenant en outre :
• envoyer, à l'unité POCT mobile (10), une série d'exécutables de contrôle de qualité préventif périodique, devant chaque exécutable de contrôle de qualité préventif périodique être exécuté pour un analyseur POCT (11, 32) identifié ayant un état lié ;
• recevoir, de l'unité POCT mobile (10), une série de contrôles de qualité préventifs périodiques achevés, comprenant chaque contrôle de qualité préventif périodique achevé le résultat du contrôle de qualité préventif périodique achevé pour son analyseur POCT (11, 32) identifié associé avec un état lié ;
• déterminer un état lié pour chaque analyseur POCT (11, 32) identifié avec contrôle de qualité préventif périodique achevé et ayant des données de qualité associées satisfaisant des exigences de qualité prédéfinies, comprenant lesdites données de qualité associées le résultat de l'au moins un contrôle de qualité préventif périodique exécuté associé, et représentant ledit état lié que l'analyseur POCT (11, 32) identifié est opérationnel pour l'unité POCT mobile (10).

13. Le procédé mis en oeuvre par ordinateur selon l'une quelconque des revendications 8 à 12, comprenant en outre :
• lorsqu'une série d'identités d'analyseurs POCT présents sont reçues de l'unité POCT mobile (10):
o vérifier, pour chaque analyseur POCT (11, 32) identifié ayant un état lié, si l'analyseur POCT (11, 32) identifié ayant un état lié n'est pas un analyseur POCT présent (11, 32), produisant ladite vérification une série d'analyseurs POCT liés mais absents (31) ;
∘ obtenir une durée d'absence accumulée pour chaque analyseur POCT lié mais absent (31) ;
∘ déterminer un état non lié pour chaque analyseur POCT lié mais absent (31) ayant une durée d'absence accumulée dépassant un seuil de durée d'absence prédéfini.

14. Une unité d'analyse au chevet du patient (POCT) mobile (10) avec profil dynamique, comprenant des moyens informatiques adaptés pour effectuer chacune des étapes de la revendication 1.

15. Un système de gestion central (20) d'analyse au chevet du patient (POCT) comprenant des moyens informatiques adaptés pour effectuer chacune des étapes de la revendication 8.

16. Un réseau d'unités d'analyse au chevet du patient (POCT) mobiles (10)avec profil dynamique, comprenant :
- un système de gestion central (20) POCT selon la revendication 15 ;
- au moins une unité POCT mobile (10) selon la revendication 14 ;
- au moins un analyseur POCT (11, 32) comprenant des moyens d'identification sans fils pour l'unité POCT mobile (10) identifiant sans fil ledit analyseur POCT (11,32).
